# EUROPÄISCHE PATENTSCHRIFT

(11) **EP 1 627 620 B1**
(45) Veröffentlichungstag und Bekanntmachung des Hinweises auf die Patenterteilung: **09.06.2010**
(21) Anmeldenummer: 05015297.4
(22) Anmeldetag: 14.07.2005
(51) Int. Cl.: A61K 6/10, A61K 6/093

(54) **Zusammensetzungen auf Basis silanterminierter Polyether und deren Verwendung**
Silane-terminated polyether compositions and their use
Compositions à base de polyether silaniques terminées et leurs utilisation

(30) Priorität: 19.08.2004 DE 102004040386
(43) Veröffentlichungstag der Anmeldung: 22.02.2006
(73) Patentinhaber: Heraeus Kulzer GmbH, 63450 Hanau (DE)
(72) Erfinder: Schaub, Matthias, Dr., 63579 Freigericht-Horbach (DE); Urbas, Holger, 47800 Krefeld (DE); Klein, Markus, 41469 Neuss (DE); Knispel, Gottfried, Dr., 51375 Leverkusen (DE)
(74) Vertreter: Kühn, Hans-Christian

(56) Entgegenhaltungen:
- EP-A- 0 269 819
- EP-A- 0 939 107
- EP-A- 1 081 191
- EP-A- 1 226 808
- EP-A- 1 402 873
- WO-A-2004/022618
- WO-A-2005/077321

## Beschreibung

Die Erfindung betrifft Zusammensetzungen auf der Basis von silanterminierten Polyetherderivaten und ihre Verwendung zur Herstellung von Abformmassen, die insbesondere im Dentalbereich Verwendung finden.

Die Herstellung von silanterminierten Polyethern bzw. Polyetherderivaten und ihre Verwendung zur Herstellung von Abformmassen ist an sich bekannt. So beschreibt EP 0 269 819 B1 die Verwendung von Abmischungen enthaltend Ether-, Urethan- und Harnstoffgruppen enthaltende Polyadditionsprodukte mit Alkoxysilanendgruppen zur Herstellung von Abform- oder Dubliermassen im Dentalbereich.

In DE 43 07 024 und DE 44 39 769 werden ähnliche Systeme offenbart, nämlich Kunststoffe mit mindestens einem Silan-, Ether- und Urethangruppen und gegebenenfalls Harnstoffgruppen enthaltenden Polyadditionsprodukt mit einer überwiegend linearen Molekülstruktur und überwiegend aliphatisch oder cycloaliphatisch gebundenen Ether oder Urethansegmenten und einem Zahlenmittel der Molmasse im Bereich von 800 - 20000, mit einem Gehalt an endständig angeordneten Silylgruppen, wobei mindestens eine Ether-Gruppe in mindestens einem der Substituenten am Siliziumatom vorliegt.

In DE 101 04 079.2-42 werden schließlich Mischungen auf Basis alkoxysilylfunktioneller Polyether mit linearer oder verzweigter Hauptkette als Abform- und Dubliermassen im Dentalbereich beschrieben.

In DE 102 44 693 sind zweikomponentige Zubereitungen auf der Grundlage silanfunktionalisierter Polyetherderivate beschrieben, die in der Basis-Komponente antacid wirkende Komponenten enthalten.

Darüber hinaus sind silanfunktionalisierte Polyetherderivate auch als Additive für die Aktivatorkomponenten von kondensationsvernetzende Silikonmassen bekannt. Derartige Systeme sind in DE 198 08 557 beschrieben.

WO2004/022618 A1 beschreibt Polymermassen auf Basis alkoxysilanterminierter Polymere, die sich durch Endgruppen der Formel A-CH2-SiR1a(OR2)3-a auszeichnen. Diese Systeme haben offenbar den Vorteil, dass sich die Härtungsgeschwindigkeit durch Zugabe spezieller Additive gut regulieren lässt.

WO2005/077321 A1 beschreibt Dentalmaterialien auf Basis alkoxysilanterminierter Polyether mit Wasser und einem Katalysator, wobei der Katalysator ein Salz ist. Damit werden offenbar lagerstabile Massen erhalten, die mit säurelabilen Zusätzen kompatibel sind.

Abformmassen auf der Grundlage silanterminierter Polyether bestehen in der Regel aus einer Katalysatorkomponente, die Wasser und organische und/oder anorganische Säuren enthält und der Basiskomponente. Die Komponenten werden getrennt gelagert und vor der Anwendung gemischt.

Bei der Verwendung als dentales Abformmaterial wird die angemischte Zubereitung im fließfähigen Zustand in den Mund des Patienten eingebracht und auf die Zahnreihe gedrückt. Innerhalb weniger Minuten bindet die Abformmasse dann zu einem elastischen Produkt ab.

Es bestehen vielfältige Anforderungen bezüglich der Eigenschaften eines Abformmaterials im fließfähigen und im elastischen Zustand.

Wesentliche Anforderungen bestehen hinsichtlich Anmischbarkeit der Einzelkomponenten, Abbindeverhalten der angemischten Abformmasse sowie Detailwiedergabe, Dimensionsgenauigkeit und mechanischen Eigenschaften des elastomeren Formkörpers.

Die Konsistenzen der Einzelkomponenten müssen so beschaffen sein, dass innerhalb weniger Sekunden eine gleichmäßige Vermischung zu einer homogenen Masse möglich ist. Dies kann beispielsweise bei sogenannten Tubenmaterialien durch Anmischen per Hand erfolgen. Bei Produkten, die in Doppelkammerkartuschen oder Schlauchbeuteln abgepackt sind, werden die beiden Komponenten durch Austraggeräte ausgefördert und über statische oder dynamische Mischer homogen angemischt.

In diesem fließfähigen Zustand muss das Material für eine zur Abdrucknahme hinreichend lange Zeit, in der Regel etwa 30s bis 4 min, verbleiben. Nach etwa 2 bis 5-minütiger Mundverweildauer soll das Produkt in einen elastomeren Zustand übergegangen sein, so dass eine Mundentnahme ohne Beeinträchtigung des Abdrucks möglich ist. Nach EN ISO 4823 werden folgende Anforderungen an das abgebundene Material gestellt:

Die Rückstellung nach Verformung muss bei mindestens 96,5 % liegen, die Detailwiedergabe mindestens 20 µm betragen und die Dimensionsänderung maximal 1,5 % betragen.

Darüber hinaus ist eine ausreichende Reißfestigkeit und Reißdehnung des elastischen Festkörpers erforderlich.

Bei den Abformmassen auf der Grundlage silanterminierter Polyetherderivate nach dem Stand der Technik erwies es sich als sehr schwierig, bei vorgegebener Verarbeitungszeit, Reißfestigkeit und Härte Produkte zu erhalten, die leicht anmischbar sind und eine hohe Reißdehnung aufweisen.

Durch die Erhöhung des mittleren Molekulargewichts der eingesetzten silanterminierten Polyether lässt sich in einem gewissen Rahmen eine Erhöhung der Reißdehnung erreichen. Allerdings weisen sowohl die dafür erforderlichen silanterminierten Polyetherderivate als auch die daraus erhaltenen Abformmassen eine sehr hohe Viskosität auf. Durch diese hohe Visksosität ist eine homogene Vermischung der Massen nur überaus schwer erreichbar.

Abformmassen, die noch eine akzeptable Anmischbarkeit aufweisen, zeichnen sich durch Viskositäten (23°C, 3s⁻¹) von < 700 Pas aus.

Entsprechende geeignete silanterminierte Polyetherderivate, aus denen solche gut anzumischenden Massen erhalten werden können, zeichnen sich durch Viskositäten (23°C, 3s-1) von < 160 Pas aus. Üblicherweise führen silanterminierte Polyetherderivate mit niedrigeren Viskositäten auch zu Abformmassen mit niedrigeren Viskositäten, die dann leichter anzumischen sind.

Aufgabe der vorliegenden Erfindung ist es daher, silanterminierte Polyetherderivate bereitzustellen, auf deren Basis Zubereitungen erhalten werden können, die bei einer Verarbeitungszeit von 60 bis 240 s nach der Aushärtung Reißfestigkeitswerte von über 1 N/mm² (bestimmt nach DIN 53504) und eine Shore A-Härte von 25 bis 80 (bestimmt nach DIN 53505) aufweisen und sich durch leichte Anmischbarkeit bzw. eine möglichst niedrige Viskosität der Einzelkomponenten sowie eine hohe Reißdehnung (bestimmt nach DIN 53504) des abgebundenen Materials auszeichnen.

Diese Aufgabe wird durch die Verwendung silanterminierter Polyetherderivate gelöst, die folgende Strukturmerkmale aufweisen:
einen Gehalt an Polyethergruppen von 10 bis 98, bevorzugt 50-90 % und
einen Gehalt von 1 bis 25% an Endgruppen der Struktur

   -NR¹X-Si(OR²)ₘR³₍₃₋ₘ₎ (I)
worin bedeuten:
R¹, R², R³: unabhängig voneinander H oder einen gesättigten oder ungesättigten, linearen, verzweigten, cyclischen oder polycyclischen Kohlenwasserstoffrest mit 0 bis 10 Heteroatomen der Gruppe O, N, S und insgesamt 1 bis 30 C-Atomen;
m 1, 2 oder 3;
X eine Gruppe der Summenformel CₙH₂ₙ;
n eine ganze Zahl ≥ 3,
   "mit der Maßgabe, dass n > 3 ist und X mindestens ein tertiäres und/oder quartäres C-Atom enthält, falls R¹ für H steht."

Die Strukturmerkmale liegen dabei im selben Molekül bzw. Makromolekül vor.
R¹, R², R³ bedeuten insbesondere unabhängig voneinander H,
- gegebenenfalls durch -O-, -S- oder -N< unterbrochenes C₁₋₃₀Alkyl, C₂₋₂₀Alkenyl oder C₂₋₂₀-Alkinyl
- gegebenenfalls durch C₁₋₆Alkyl, C₂₋₆Alkenyl, oder C₁₋₆Alkoxy substituiertes C₁₋₁₂Cycloalkyl, C₁₋₁₀Aryl oder C₁₋₉Heteroaryl oder
- C₇₋₁₂Aralkyl oder C₇₋₁₂Alkaryl.

Die Erfindung betrifft somit Zusammensetzungen enthaltend mindestens eines dieser beschriebenen silanterminierten Polyetherderivate sowie Wasser und mindestens eine organische oder anorganische Säure.

Vorzugsweise bedeutet der Rest X eine lineare oder verzweigte Gruppe der Summenformel CₙH₂ₙ, mit n > 3, und mindestens einem tertiären und/oder mindestens einem quartären C-Atom.

Für die Alkylreste R¹, R², R³ kommen alle geradkettigen und verzweigten Reste mit 1 - 30 C-Atomen in Betracht, wie z. B. Methyl, Ethyl, Propyl, Isopropyl, Butyl, sek.-Butyl, tert.-Butyl, Isobutyl, Pentyl, Isopentyl, Neopentyl, Hexyl, Isohexyl, Heptyl, Octyl, 2-Ethylhexyl, Nonyl, Decyl, Undecyl, Dodecyl, Octadecyl, Eicosyl, Quatrodecyl, Tridecyl, 1-methylbutyl, 2-methylbutyl, 3-methylbutyl, 2,2-dimethylpropyl, 1-ethylpropyl, 1,1-dimethylpropyl, 1,2-dimethylpropyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl, 1-ethyl-2-methylpropyl, Tridecyl, Tetradecyl, Pentadecyl, Hexadecyl, Heptadecyl, Nonadecyl. Bevorzugte Reste sind davon für R¹ die mit 1-15 C-Atomen, für R² und R³ die mit 1 - 10 C-Atomen.

Als C₂₋₂₀Alkenyl kommen z.B. Vinyl, Allyl (Propenyl), Isopropenyl, 1-Butenyl, 2-Butenyl, 1-Pentenyl, 2-Pentenyl, 2-Methyl-1-butenyl, 2-Methyl-2-butenyl, 3-Methyl-1-butenyl, 1-Hexenyl, 2-Hexenyl, 1-Heptenyl, 2-Heptenyl, 1-Octenyl, 2-Octenyl, Decenyl, Oleyl, -, Elaidyl-, Ricinol-, Linoleyl-, Linolenyl-, Gadoleyl-, Arachidyl- oder Erucyl in Frage.

Als C₃₋₂₀Alkinyl kommen z.B. Propinyl, 3-Butinyl, 2-Butinyl, n-2-Octinyl und n-2-Octadecinyl in Frage.

Bevorzugt werden davon die C₂₋₅Alkenyl- und C₃₋₄Alkinylgruppen.

Als Cycloalkylreste sind z.B. Cyclopentyl, Cyclohexyl Cyclooctyl, Cyclododecyl gemeint.

Für die Alkoxygruppen kommen geradkettige oder verzweigte Reste mit 1-6 C-Atomen wie z. B. Methoxy, Ethoxy, Propoxy, Isopropoxy, Butoxy, Isobutoxy, sek.-Butoxy, tert.-Butoxy in Frage. Bevorzugt werden Alkoxygruppen mit 1-4 C-Atomen.

Mit den Arylgruppen sind etwa Phenyl, 1-Naphthyl oder 2-Naphthyl gemeint.

Die Aralkylreste R und R mit 7-12 C-Atomen können z. B. Benzyl, Phenethyl, 2-Phenethyl, 3-Phenylpropyl, 1-Naphthylmethyl sein.

Als Alkarylreste kommen etwa Toluyl oder Mesityl in Frage.

Als Alkylengruppe X kommen geradkettige oder verzweigtkettige, gesättigte Alkylenreste in Frage, Beispielsweise seien genannt: Ethylethylen, Tetramethylen, Pentamethylen, 1-Methyltetramethylen, 1-Methyltrimethylen, 1,1-Trimethylenethylen, 1,1-Tetramethylenmethylen, n-Propylen, 1-Methylethylen, n-Butylen, 1-Methylpropylen, 2-Methylpropylen, 1,1-Dimethylethylen, n-Pentylen, 1-Methylbutylen, 2-Methylbutylen, 3-Methylbutylen, 2,2-Dimethylpropylen, 1-Ethylpropylen, n-Hexylen, 1,1-Dimethylpropylen, 1,2-Dimethylpropylen, 1-Methylpentylen, 2-Methylpentylen, 3-Methylpentylen, 4-Methylpentylen, 1,1-Dimethylbutylen, 1,2-Dimethylbutylen, 1,3-Dimethylbutylen, 2,2-Dimethylbutylen, 2,3-Dimethylbutylen, 3,3-Dimethylbutylen, 1-Ethylbutylen, 2-Ethylbutylen, 1,1,2-Trimethylpropylen, 1,2,2-Trimethylpropylen, 1-Ethyl-1-methylpropylen, 1-Ethyl-2-methylpropylen, n-Heptylen, n-Octylen, n-Nonylen, n-Decylen, n-Undecylen, n-Dodecylen, Hexadecylen oder Octadecylen.

Die Herstellung dieser silanterminierten Polyetherderivate erfolgt in bevorzugter Weise analog zu dem in EP 0 269 819 B1 beschriebenen Verfahren durch Umsetzung von OH-funktionellen Polyethern mit zwei oder mehr OH-Funktionen mit aliphatischen und/oder cycloaliphatischen Diisocyanaten, wobei gegebenenfalls weitere OH-funktionelle Verbindungen, wie Alkohole, Polyesterpolyole, Polyolefinpolyole, Polycarbonatpolyole oder OH-terminierte Polysiloxane zugesetzt werden und die erhaltenen isocyanatfunktionellen Polyetherderivate anschließend mit geeigneten Aminoalkylalkoxysilanen und gegebenenfalls aliphatischen und/oder cycloaliphatischen Diaminen mit primären und/oder sekundären Aminogruppen umgesetzt werden.

Die Herstellung der erfindungsgemäß eingesetzten Polyetherderivate kann auch durch Umsetzung von OH-funktionellen Polyethern mit zwei oder mehr OH-Funktionen mit Isocyanatoalkylalkoxysilanen erfolgen.

Die zur Herstellung der erfindungsgemäß eingesetzten silanterminierten Polyetherderivate verwendeten OH-funktionellen Polyether sind z.B. Homo- oder Copolymerisationsprodukte von Epoxiden wie Ethylenoxid, Propylenoxid, Butylenoxid, Tetrahydrofuran, Styroloxid oder Epichlorhydrin, mit OH-Funktionalitäten von zwei bis zehn, bevorzugt zwei bis sechs und einem Zahlenmittel des Molekulargewichts von 800 bis 20000, wie sie vielfach in der Literatur beschrieben sind.

Geeignete Diisocyanate sind insbesondere solche mit aliphatisch und/oder cycloaliphatisch gebundenen Isocyanatgruppen der Formel Q(NCO)₂ in welcher Q für einen aliphatischen Kohlenwasserstoffrest mit 2 bis 12 Kohlenstoffatomen oder einen cycloaliphatischen bzw. gemischt aliphatisch-cycloaliphatischen Kohlenwasserstoffrest mit 4 bis 14 Kohlenstoffatomen steht.

Beispiele derartiger Diisocyanate sind Ethylendiisocyanat, Tetramethylenddisocyanat, Hexamethylendüsocyanat, Dodecamethylendüsocyanat, Cyclobutan-1,3-diisocyanat, Cyclohexan-1,3- und 1,4-diisocyanat oder 1-Isocyanato-3,3,5-trimethyl-5-isocyanatomethyl-cyclohexan bzw. beliebige Gemische derartiger Diisocyanate. Vorzugsweise werden zur Herstellung der erfindungsgemäß eingesetzten Polyetherderivate cycloaliphatische bzw. gemischt aliphatisch-cycloaliphatische Diisocyanate eingesetzt. Besonders bevorzugt ist 1-Isocyanato-3,3,5-trimethyl-5-isocyanato-methyl-cyclohexan (Isophorondiisocyanat).

Geeignete Isocyanatoalkylalkoxysilane zeichnen sich durch folgende Struktur aus:

OCN-X-Si(OR²)ₘR³₍₃₋ₘ₎,

m =1, 2 oder 3,
geeignete Aminoalkylalkoxysilane zeichnen sich durch folgende Struktur aus:

HNR¹-X-Si(OR²)ₘR³₍₃₋ₘ₎,

m =1, 2 oder 3,
worin bedeuten
R¹, R², R³: unabhängig voneinander H oder einen gesättigten oder ungesättigten, linearen, verzweigten, cyclischen oder polycyclischen Kohlenwasserstoffrest mit 0 bis 10 Heteroatomen der Gruppe O, N, S und insgesamt 1 bis 30 C-Atomen,
X eine Gruppe der Summenformel CₙH₂ₙ, mit n ≥ 3, vorzugsweise 3 ≤ n ≤ 20, mit der Maßgabe, dass n > 3 ist, falls R¹ = H.

Vorzugsweise bedeuten R¹ H oder einen Kohlenwasseratoffrest mit 1 bis 15 C-Atomen, wie z.B. -methyl, -ethyl, -n-propyl, -i-propyl, -n-butyl, n-pentyl, n-octyl, 2-ethyl-hexyl, phenyl, benzyl und R² und R³ einen Kohlenwasserstoffrest mit 1 bis 10 C-Atomen, z.B. -methyl, -ethyl, -n-propyl, -i-propyl, -n-butyl. In einer speziellen Ausführung kann R² einen Rest aus Oxyalkyl-alkylgruppen bedeuten, wie z.B. in -CH₂CH₂-O-CH₃, -CH₂CH₂-O-CH₂CH₂-O-CH₃ oder in

-CH₂CHCH₃-O-CH₂CHCH₃-O-CH₃.

Beispiele sind
Aminoalkyltrialkoxysilane, wie 4-Aminobutyltrimethoxysilan,
N-Alkylaminopropylalkoxysilane, wie N-Methylaminopropyltrimethoxysilan,
N-Methylaminopropyltriethoxysilan, N-Methylaminopropylmethyldimethoxysilan oder N-ethylaminopropyltrimethoxysilan,
4-Amino-3,3-dimethylbutyltrimethoxysilan,
N-Ethyl-aminoisobutyltrimethoxysilan,
N-Phenylaminopropyltrimethoxysilan,
N-Cyclohexylaminopropyltrimethoxysilan,
N-methylaminopropyltris(methoxyethoxyethoxy)silan,
4-Aminobutyltris(methoxyethoxyethoxy)silan.

Geeignete Diamine zur Herstellung der erfindungsgemäß eingesetzten Polyetherderivate sind primäre oder sekundäre Aminogruppen aufweisende aliphatische, cycloaliphatische oder gemischt aliphatisch-cycloaliphatische Diamine des Molekulargewichts 60 bis 5000. Beispiele sind Ethylendiamin, Tetramethylendiamin, Hexamethylendiamin, 4,4'-Diamino-dicyclohexylmethan, 1,4-Diaminocyclohexan, 4,4'-Diamino, 3,3'-dimethyl-dicyclohexylmethan, 1-Amino, 3,3,5-trimethyl-5-aminomethylcyclohexan oder Polypropylenoxid-Diamine.

Die Herstellung der erfindungsgemäß eingesetzten silanterminierten Polyetherderivate kann ohne die Verwendung von zinnhaltigen Katalysatoren, insbesondere organischen Zinnverbindungen erfolgen.

Erfindungsgemäße Zusammensetzungen (bzw. Mischungen), die auf Basis der beschriebenen silanterminierten Polyetherderivate erhalten werden, bestehen in der Regel aus:
(A) 2 bis 85 %, bevorzugt 15 bis 40 % silanterminiertes Polyetherderivat mit Endgruppen der Struktur NR¹-X-Si(OR²)ₘR³₍₃₋ₘ₎, mit den oben angegebenen Bedeutungen für R¹, R², R³, X und m,
(B) Abmischungen von Wasser mit organischer und/oder anorganischer Säure,
(C) 5 bis 70 %, bevorzugt 15 bis 50 % Verdünner,
(D) 10 bis 90 %, bevorzugt 20 bis 70 % Füllstoffe,
(E) 0 bis 10 %, bevorzugt 0 bis 8 % Additive, wie Farbstoffe, Pigmente, Aromen, Stabilisatoren, Verzögerer, Emulgatoren.

Als Bestandteil (C) kommen insbesondere niedermolekulare Verbindungen oder bei Raumtemperatur flüssige Polymerein Frage.

Beispiele dafür sind Glycerin, Phthalsäureester, Citronensäureester, aromatische Kohlenwasserstoffe, wie z.B. Dibenzyltoulol, aromatische und aliphatische Sulfonsäureester oder bei Raumtemperatur flüssige Polyether, z.B. solche, wie sie zur Herstellung von Komponente (A) eingesetzt werden, bzw. beliebige Abmischungen daraus. Dabei ist die Verwendung von aromatischen Kohlenwasserstoffen und von Polyethern bevorzugt.

Gemäß Bestandteil (D) werden z.B. anorganische Füllstoffe mit unbeladener oder beladener Oberfläche eingesetzt, wie z.B. Quarz- oder Cristobalitmehle, gefällte oder pyrogene Kieselsäuren. Organische Füllstoffe wie hydrierte Rizinusöle oder Rizinusölderivate, Polyamide, Polyester, Paraffine, Wachse, Fette können ebenfalls eingesetzt werden.

Gemäß Bestandteil (B) werden Mischungen von Wasser mit organischen Säuren oder mit anorganischen Säuren eingesetzt. Die Geschwindigkeit der Abbindereaktion ist abhängig von der Säurestärke der eingesetzten Säure. Verwendbar sind z.B. Salzsäure, Phosphorsäure, Hexafluorophosphorsäure, Hexafluoroantimonsäure, Tetrafluoroborsäure, 4-Toluolsulfonsäure, Benzolsulfonsäure4-Brombenzolsulfonsäure, 4-Chlorbenzolsulfonsäure, Alkansulfonsäuren, Carbonsäuren, wie Essigsäure, Propionsäure, Bernsteinsäure, Weinsäure, Trimellitsäure, Benzoesäure, Phenylessigsäure, Zitronensäure, Maleinsäure, Adipinsäure.

Mischungen von Wasser mit organischen und/oder anorganischen Säuren im Gewichtsverhältnis 1 : 0,01 bis 1 : 40 werden bevorzugt eingesetzt.

Bevorzugt sind als Säuren Sulfonsäuren, besonders bevorzugt 4-Toluolsulfonsäure, wobei die Mischung mit Wasser vorzugsweise zwischen 5 und 50 % 4-Toluolsulfonsäure enthält.

Die erfindungsgemäßen Zusammensetzungen enthalten gemäß Bestandteil (E) 0 - 10 % an weiteren Additiven, wie Farbstoffe, Pigmente, Aromastoffe, Thixotropiemittel, Stabilisatoren wie z.B. Silane und antacid wirkende Verbindungen wie z.B. Amine oder Aminosilane, Emulgatoren oder sonstigen, üblichen Additiven.

In der Regel werden die beschriebenen Mischungen als zweikomponentige Materialien formuliert, bestehend aus einer Basiskomponente und einer Katalysatorkomponente, wobei die Basiskomponente den Bestandteil (A) und gegebenenfalls die Bestandteile (C), (D) und (E) enthält und die Katalysatorkomponente den Bestandteil (B) und gegebenenfalls die Bestandteile (C), (D) und (E) enthält. Dabei werden Basis- und Katalysatorkomponente üblicherweise so eingestellt, dass eine Abmischung im Volumenverhältnis von Basis- zu Katalysatorkomponente von 1:1 bis 1:10 erfolgt.

Die erfindungsgemäßen Zusammensetzungen werden üblicherweise zur Abformung, bevorzugt zu zahnmedizinischen oder zahntechnischen Zwecken, eingesetzt. Besonders bevorzugt ist die einzeitige einphasige oder mehrphasige zahnmedizinische Abdrucknahme und die Bissregistrierung.

Gegenstand der Erfindung sind auch Behältnisse und Mischvorrichtungen, enthaltend aus den erfindungsgemäßen Zusammensetzungen hergestellte Massen, wie z.B. Kartuschen, Schlauchbeutel, statische und dynamische Mischer bzw. Mischgeräte.

Die Erfindung wird anhand der folgenden Beispiele näher erläutert, ohne dass sie auf diese beschränkt werden soll. Prozentangaben beziehen sich, sofern nicht anders angegeben, auf das Gewicht - wie auch in der übrigen Beschreibung.

### Beispiele

Synthese linearer silanterminierter Polyetherderivate mit einem mittleren Molekulargewicht von ca. 4000

### SPEA1

700g (0,233 mol) eines linearen Polypropylenoxiddiols mit einem mittlerem Molekulargewicht von ca. 3000 werden für 30 Minuten bei 120 °C bei 5 mbar entwässert. Danach werden 103,5g (0,466 mol) Isophorondiisocyanat (im folgenden IPDI genannt) zugefügt und für 4 Stunden bei 120 bis 140 °C unter Stickstoff gerührt. Der Isocyanatgehalt des Prepolymeren wird mit 2,25 % bestimmt. Zu dem Prepolymeren werden bei 30 °C unter Rühren und Stickstoffatmosphäre 95,25g (0,43 mol) N-Ethylaminoisobutyltrimethoxysilan zugesetzt. Nach weiterem Rühren für 60 min bei 60 °C erhält man nach Abkühlen ein klares farbloses Produkt mit einer Viskosität bei 23 °C von 30 Pas.

### SPEA2

700g (0,233 mol) eines linearen Polypropylenoxxiddiols mit einem mittlerem Molekulargewicht von ca. 3000 werden für 30 Minuten bei 120 °C bei 5 mbar entwässert. Danach werden 103,5g (0,466 mol) IPDI zugefügt und für 4 Stunden bei 120 bis 140 °C unter Stickstoff gerührt. Der Isocyanatgehalt des Prepolymeren wird mit 2,15 % bestimmt.

Zu dem Prepolymeren werden bei 30 °C unter Rühren und Stickstoff 88,5g (0,4 mmol) 4-Amino-3,3-dimethylbutyltrimethoxysilan zugesetzt. Nach weiterem Rühren für 60 min bei 60 °C erhält man nach Abkühlen ein klares farbloses Produkt mit einer Viskosität bei 23 °C von 133 Pas.

### SPEA3 (Vergleichsbeispiel)

700g (0,233 mol) eines linearen Polypropylenoxxiddiols mit einem mittlerem Molekulargewicht von ca. 3000 werden für 30 Minuten bei 120 °C bei 5 mbar entwässert. Danach werden 103,5g (0,466 mol) IPDI zugefügt und für 4 Stunden bei 120 bis 140 °C unter Stickstoff gerührt. Der Isocyanatgehalt des Prepolymeren wird mit 2,20 % bestimmt.

Zu dem Prepolymeren werden bei 30 °C unter Rühren und Stickstoff 90,7g (0,41 mol) Aminopropyltriethoxysilan zugesetzt. Nach weiterem Rühren für 60 min bei 60 °C erhält man nach Abkühlen ein klares farbloses Produkt mit einer Viskosität bei 23 °C von 105 Pas.

Synthese linearer silanterminierter Polyetherderivate mit einem mittleren Molekulargewicht von 5500.

### SPEB1

700g (0,233 mol) eines linearen Polypropylenoxiddiols mit einem mittlerem Molekulargewicht von ca. 3000 werden für 30 Minuten bei 120 °C bei 5 mbar entwässert. Danach werden 86,46g (0,39 mol) IPDI zusammen mit 0,05g Dibutylzinndilaurat zugefügt und für 3 Stunden bei 100°C unter Stickstoff gerührt. Der Isocyanatgehalt des Prepolymeren wird mit 1,65 % bestimmt. Zu dem Prepolymeren werden bei 30°C unter Rühren und Stickstoff 67,38g (0,3 mol) N-Ethylaminoisobutyltrimethoxysilan zugesetzt. Nach weiterem Rühren für 60 min bei 60 °C erhält man nach Abkühlen ein klares farbloses Produkt mit einer Viskosität bei 23 °C von 32 Pas.

### SPEB2

700g (0,233 mol) eines linearen Polypropylenoxiddiols mit einem mittlerem Molekulargewicht von ca. 3000 werden für 30 Minuten bei 120 °C bei 5 mbar entwässert. Danach werden 86,46g (0,39 mol) IPDI zusammen mit 0,05g Dibutylzinndilaurat zugefügt und für 3 Stunden bei 100 °C unter Stickstoff gerührt. Der Isocyanatgehalt des Prepolymeren wird mit 1,65 % bestimmt. Zu dem Prepolymeren werden bei 30°C unter Rühren und Stickstoff 70,6g (0,3 mol) N-Butylaminopropyltrimethoxysilan zugesetzt. Nach weiterem Rühren für 60 min bei 60 °C erhält man nach Abkühlen ein klares farbloses Produkt mit einer Viskosität bei 23 °C von 42 Pas.

### SPEB3 (Vergleichsbeispiel)

700g (0,233 mol) eines linearen Polypropylenoxiddiols mit einem mittlerem Molekulargewicht von ca. 3000 werden für 30 Minuten bei 120 °C bei 5 mbar entwässert. Danach werden 86,46g (0,39 mol) IPDI zusammen mit 0,05g Dibutylzinndilaurat zugefügt und für 3 Stunden bei 100°C unter Stickstoff gerührt. Der Isocyanatgehalt des Prepolymeren wird mit 1,65 % bestimmt. Zu dem Prepolymeren werden bei 30 °C unter Rühren und Stickstoff 66,4g (0,3 mol) Aminopropyltriethoxysilan zugesetzt. Nach weiterem Rühren für 60 min bei 60°C erhält man nach Abkühlen ein klares farbloses Produkt mit einer Viskosität bei 23 °C von 116 Pas.

Synthese linearer silanterminierter Polyetherderivate mit einem mittleren Molekulargewicht von 7000.

### SPEC1

700g (0,233 mol) eines linearen Polypropylenoxiddiols mit einem mittlerem Molekulargewicht von ca. 3000 werden für 30 Minuten bei 120 °C bei 5 mbar entwässert. Danach werden 77,4g (0,35 mol) IPDI zusammen mit 0,05g Dibutylzinndilaurat zugefügt und für 3 Stunden bei 100°C unter Stickstoff gerührt. Der Isocyanatgehalt des Prepolymeren wird mit 1,15 % bestimmt. Zu dem Prepolymeren werden bei 30 °C unter Rühren und Stickstoff 46,5g (0,21 mol) N-Ethylaminoisobutyltrimethoxysilan zugesetzt. Nach weiterem Rühren für 60 min bei 60 °C erhält man nach Abkühlen ein klares farbloses Produkt mit einer Viskosität bei 23 °C von 80 Pas.

### SPEC2 (Vergleichsbeispiel)

700g (0,233 mol) eines linearen Polypropylenoxiddiols mit einem mittlerem Molekulargewicht von ca. 3000 werden für 30 Minuten bei 120 °C bei 5 mbar entwässert. Danach werden 77,4g (0,35 mol) IPDI zusammen mit 0,05g Dibutylzinndilaurat zugefügt und für 3 Stunden bei 100°C unter Stickstoff gerührt. Der Isocyanatgehalt des Prepolymeren wird mit 1,15 % bestimmt. Zu dem Prepolymeren werden bei 30 °C unter Rühren und Stickstoff 46,5g (0,21 mol) Aminopropyltriethoxysilan zugesetzt. Nach weiterem Rühren für 60 min bei 60 °C erhält man nach Abkühlen ein klares farbloses Produkt mit einer Viskosität bei 23 °C von 165 Pas.

**Tab. 1: Eigenschaften der erfindungsgemäß eingesetzten silanterminierten Polyetherderivate SPEA1, SPEA2, SPEB1, SPEB2 und SPEC1 sowie der in Vergleichsbeispielen eingesetzten silanterminierten Polyetherderivate SPEA3, SPEB3 und SPEC2:**

| | SPEA1 | SPEA2 | SPEA3 | SPEB1 | SPEB2 | SPEB3 | SPEC1 | SPEC2 | |
|---|---|---|---|---|---|---|---|---|---|
| | | | | | | | | | |
| mittleres Molekulargewicht ca | 4000 | 4000 | 4000 | 5500 | 5500 | 5500 | 7000 | 7000 | |
| Gehalt an Poly ethergruppen [%] | 78,0 | 78,5 | 78,3 | 82,0 | 81,7 | 82 | 85 | 85 | |
| Gehalt an End gruppen der Struktur NR'-X- Si(OR²)ₘ R³₍₃₋ₘ₎, wobei X fürCₙH₂ₙ, steht [%] | 10,6 | 9,9 | 10,1 | 7,9 | 8,2 | 7,8 | 5,6 | 5,6 | |
| n | 4 | 6 | 3 | 4 | 3 | 3 | 4 | 3 | |
| R¹ | C₂H₅ | H | H | C₂H₅ | C₄H₉ | H | C₂H₅ | H | |
| Viskosität (23°C, 3s⁻¹) [Pas] | 30 | 133 | 105 | 32 | 42 | 116 | 80 | 165 | |

### Herstellung der Basiskomponenten:

In einem Labordissolver werden die Bestandteile der Basiskomponenten (Zusammensetzung s. Tab. 2) für 3 Stunden bei einem Druck < 50 mbar zu einer homogenen pastösen Masse gemischt.

**Tab 2: Prozentuale Zusammensetzungen und Viskositäten der erfindungsgemäßen Basiskomponenten BA1, BA2, BB1, B2 und BC. Vergleichsbeispiele: VBA, VBB, VBC**

| | | BA1 | BA2 | VBA | BB1 | BB2 | VBB | BC | VBC |
|---|---|---|---|---|---|---|---|---|---|
| SPEA1 | | 20 | | | | | | | |
| SPEA2 | | | 20 | | | | | | |
| SPEA3 | | | | 20 | | | | | |
| SPEB1 | | | | | 20 | | | | |
| SPEB2 | | | | | | 20 | | | |
| SPEB3 | | | | | | | 20 | | |
| SPEC1 | | | | | | | | 20 | |
| SPEC2 | | | | | | | | | 20 |
| Dibenzyltoluol | | 20 | 20 | 20 | 20 | 20 | 20 | 20 | 20 |
| Quarzmehl | | 56 | 56 | 56 | 56 | 56 | 56 | 56 | 56 |
| gehärtetes Ricinusöl | | 4 | 4 | 4 | 4 | 4 | 4 | 4 | 4 |
| | | | | | | | | | |
| Viskosität (3s⁻¹, 23°C) | | 304 | 319 | 480 | 350 | 374 | 593 | 225 | 615 |

### Herstellung der Katalysatorkomponente:

Die Herstellung der Katalysatorkomponente erfolgt gemäß DE 101 04 079 A1, Beispiel 3. Die verschiedenen Basis-Komponenten werden mit der Katalysatorkomponente jeweils im Gewichts-Verhältnis 5:1 vermischt. An den Abmischungen werden die Verarbeitungszeiten (nach DIN EN ISO 4823), Härten nach Shore A (nach DIN 5305), Reißfestigkeiten und Reißdehnungen (nach DIN 53504) bestimmt. Die erfindungsgemäßen Zusammensetzungen weisen jeweils innerhalb der Serien A, B und C eine deutlich höhere Reißdehnung auf als die jeweiligen Vergleichsbeispiele.

Tab. 3: Eigenschaften der erfindungsgemäßen Abmischungen A1, A2, B1, B2 und C; VA, VB, VC: Vergleichsbeispiele

| | | Serie A | | | | Serie B | | | | Serie C | |
|---|---|---|---|---|---|---|---|---|---|---|---|
| | | A1 | A2 | VA | | B1 | B2 | VB | | C | VC |
| Verarbeitungszeit | | 1,55 | 1,75 | 2,3 | | 2,0 | 2,3 | 2,2 | | 2,6 | 2,8 |
| Shore A (1h) | | 50 | 62 | 64 | | 54 | 50 | 58 | | 50 | 55 |
| Reißfestigkeit | | 2,2 | 2,9 | 2,5 | | 3,2 | 1,7 | 2,4 | | 2,8 | 2,4 |
| Reißdehnung [%] | | 76 | 61 | 44 | | 86 | 87 | 57 | | 113 | 73 |

## Patentansprüche

1. Zusammensetzungen enthaltend
(A) mindestens ein silanterminiertes Polyetherderivat, **gekennzeichnet durch**
- einen Gehalt an Polyethergruppen von 10 bis 98% und
- einen Gehalt von 1 bis 25% an Endgruppen der Struktur
-NR¹-X-Si(OR²)ₘR³₍₃₋ₘ₎ (I)
worin bedeuten
m: 1, 2 oder 3
R¹, R², R³: unabhängig voneinander H oder einen gesättigten oder ungesättigten, linearen, verzweigten, cyclischen oder polycyclischen Kohlenwasserstoffrest mit 0 bis 10 Heteroatomen der Gruppe O, N, S und insgesamt 1 bis 30 C-Atomen,
X: eine Gruppe der Summenformel CₙH₂ₙ,
n: eine ganze Zahl ≥ 3,
mit der Maßgabe, dass n > 3 ist und X mindestens ein tertiäres und/oder quartäres C-Atom enthält, falls R¹ für H steht,
(B1) Wasser und
(B2) mindestens eine organische oder anorganische Säure.

2. Zusammensetzungen nach Anspruch 1, wobei der genannte Kohlenwasserstoffrest R¹, R² oder R³
- gegebenenfalls durch -O-, -S- oder -N< unterbrochenes C₁₋₃₀Alkyl, C₂₋₂₀Alkenyl oder C₂₋₂₀-Alkinyl,
- gegebenenfalls durch C₁₋₆Alkyl, C₂₋₆Alkenyl, oder C₁₋₆Alkoxy substituiertes C₁₋₁₂Cycloalkyl, C₁₋₁₀Aryl, C₁₋₉Heteroaryl,
- C₇₋₁₂Aralkyl oder C₇₋₁₂Alkaryl bedeutet.

3. Zusammensetzungen nach einem der vorstehenden Ansprüche, wobei (B) die Komponenten B1 und B2 als Mischung vorliegen.

4. Zusammensetzungen nach mindestens einem der vorstehenden Ansprüche, **dadurch gekennzeichnet, dass** Komponente (A) einen Gehalt an Polyethergruppen von 50 bis 90 % aufweist.

5. Zusammensetzungen nach mindestens einem der vorstehenden Ansprüche, **dadurch gekennzeichnet, dass** Komponente (A) Endgruppen der Struktur NR¹-X-Si(OR²)ₘR³₍₃₋ₘ₎, mit m =1, 2 oder 3 aufweist, wobei X eine Gruppe der Summenformel CₙH₂ₙ, mit n > 3 bedeutet und R¹, R² und R³ die in Anspruch 1 oder 2 angegebene Bedeutung haben.

6. Zusammensetzungen nach mindestens einem der vorstehenden Ansprüche 1 bis 5, **dadurch gekennzeichnet, dass** das enthaltene silanterminierte Polyetherderivat Endgruppen der Struktur NR¹-X-Si(OR²)ₘR³₍₃₋ₘ₎, mit m =1, 2 oder 3 aufweist, wobei X eine Gruppe der Summenformel CₙH₂ₙ, mit 3 ≤ n ≤ 20 und mindestens einem tertiären und/oder mindestens einem quartären C-Atom bedeutet und R¹, R² und R³ die in Anspruch 1 oder 2 angegebene Bedeutung haben.

7. Zusammensetzungen nach mindestens einem der Ansprüche 1 bis 5, **dadurch gekennzeichnet, dass** das enthaltene silanterminierte Polyetherderivat Endgruppen der Struktur NR¹-X-Si(OR²)mR³₍₃₋ₘ₎, mit m =1, 2 oder 3 aufweist, wobei X eine Gruppe der Summenformel CₙH₂ₙ, mit n ≥ 3 bedeutet,
R¹ einen Alkyl-, Cycloalkyl-, Phenyl-, Benzyl- oder Carbamat-Rest bedeutet
und R² und R³ die in Anspruch 1 oder 2 angegebene Bedeutung haben.

8. Zusammensetzungen nach mindestens einem der vorstehenden Ansprüche, **dadurch gekennzeichnet, dass** Komponente (B) Mischungen enthaltend Wasser und organische und/oder anorganische Säuren in Gewichtsmengenverhältnissen von 1 : 0,01 bis 1 : 40 aufweist.

9. Zusammensetzungen nach mindestens einem der vorstehenden Ansprüche, **dadurch gekennzeichnet, dass** als weitere Komponenten
(C) 5 bis 70% Verdünner und/oder
(D) 10 bis 90% Füllstoffe und /oder
(E) 0 bis 10% übliche Additive
enthalten sind.

10. Zusammensetzungen nach mindestens einem der vorstehenden Ansprüche, **dadurch gekennzeichnet, dass** als weitere Komponenten
(C) 15 bis 50% Verdünner und/oder
(D) 20 bis 70% Füllstoffe und/oder
(E) 0 bis 8% übliche Additive
enthalten sind.

11. Zusammensetzungen nach Anspruch 9 oder 10, wobei die Additive der Komponente (E) den Gruppen der Farbstoffe, Pigmente, Aromen, Stabilisatoren, Verzögerer, Emulgatoren angehören.

12. Verwendung von Zusammensetzungen gemäß mindestens einem der Ansprüche 1 bis 11 zur Herstellung von Dentalmassen.

13. Verwendung von Zusammensetzungen gemäß mindestens einem der Ansprüche 1 bis 11 zur Abformung.

14. Behältnis enthaltend mindestens eine Zusammensetzung gemäß mindestens einem der Ansprüche 1 bis 11.

15. Mischvorrichtung, enthaltend mindestens eine Zusammensetzung gemäß mindestens einem der Ansprüche 1 bis 11.

## Claims

1. Compositions containing
(A) at least one silane-terminated polyether derivative, **characterized by**
- a polyether group content from 10 to 98% and
- a 1 to 25% content of terminal groups having the structure
-NR¹-X-Si(OR²)ₘR³₍₃₋ₘ₎, (I)
with
m: 1, 2 or 3
R¹, R², R³: independently of each other denote H or a saturated or unsaturated, linear, branched, cyclic or polycyclic hydrocarbon residue having 0 to
10 heteroatoms from the group of O, N, S and a total of 1 to 30 carbon atoms;
X: a group with the empirical formula CₙH₂ₙ;
n: an integer ≥ 3;
provided n > 3 and X contains at least one tertiary and/or quarternary C atom, if R¹ denotes H;
(B1) water; and
(B2) at least one organic or inorganic acid.

2. Compositions according to claim 1, whereby said hydrocarbon residue R¹, R², or R³ means
- C₁₋₃₀ alkyl, C₂₋₂₀ alkenyl or C₂₋₂₀ alkinyl, possibly interrupted by -O-, -S- or - N<, if applicable;
- C₁₋₁₂ cycloalkyl, C₁₋₁₀ aryl, C₁₋₉ heteroaryl, possibly substituted by C₁₋₆ alkyl, C₂₋₆ alkenyl or C₁₋₆ alkoxy, if applicable;
- C₇₋₁₂ aralkyl or C₇₋₁₂ alkaryl.

3. Compositions according to any one of the preceding claims, whereby (B) components B1 and B2 are present in the form of a mixture.

4. Compositions according to at least one of the preceding claims, **characterized in that** component (A) has a content of polyether groups from 50 to 90%.

5. Compositions according to at least one of the preceding claims, **characterized in that** component (A) comprises terminal groups of structure NR¹-X-Si(OR²)ₘR³_{(3-m),} with m = 1, 2 or 3, whereby X is a group with empirical formula CₙH₂ₙ with n > 3, and R¹, R², and R³ have the meaning specified in claim 1 or 2.

6. Compositions according to at least one of the preceding claims 1 to 5, **characterized in that** the silane-terminated polyether derivative contained therein comprises terminal groups of structure NR¹-X-Si(OR²)ₘR³₍₃₋ₘ₎, with m = 1, 2 or 3, whereby X is a group with empirical formula CₙH₂ₙ with 3 ≤ n ≤ 20 and at least one tertiary and/or at least one quaternary C atom, and R¹, R², and R³ have the meaning specified in claim 1 or 2.

7. Compositions according to at least one of the preceding claims 1 to 5, **characterized in that** the silane-terminated polyether derivative contained therein comprises terminal groups of structure NR¹-X-Si(OR²)ₘR³₍₃₋ₘ₎, with m = 1, 2 or 3,
whereby X is a group with empirical formula CₙH₂ₙ with n ≥ 3,
R¹ is an alkyl, cycloalkyl, phenyl, benzyl or carbamate residue,
and R² and R³ have the meaning specified in claim 1 or 2.

8. Compositions according to at least one of the preceding claims, **characterized in that** component (B) comprises mixtures containing water and organic and/or inorganic acids at weight ratios from 1 : 0.01 to 1 : 40.

9. Compositions according to at least one of the preceding claims, **characterized in that**
(C) 5 to 70% diluent and/or
(D) 10 to 90% fillers and/or
(E) 0 to 10% common additives
are contained therein as further components.

10. Compositions according to at least one of the preceding claims, **characterized in that**
(C) 15 to 50% diluent and/or
(D) 20 to 70% fillers and/or
(E) 0 to 8% common additives
are contained therein as further components.

11. Compositions according to claim 9 or 10, whereby the additives of component (E) belong to the group of dyes, pigments, flavors, stabilizers, retarders, emulsifiers.

12. Use of compositions according to at least one of the claims 1 to 11 for the production of dental materials.

13. Use of compositions according to at least one of the claims 1 to 11 for taking impressions.

14. Container containing at least one composition according to at least one of the claims 1 to 11.

15. Mixing device, containing at least one composition according to at least one of the claims 1 to 11.

## Revendications

1. Compositions contenant
(A) au moins un dérivé de polyéther silane-terminé, **caractérisées par**
- une teneur en groupes polyéther de 10 à 98 % et
- une teneur de 1 à 25 % en groupes terminaux de la structure
-NR¹-X-Si(OR²)ₘR³₍₃₋ₘ₎ (I)
dans laquelle
m : représente 1, 2 ou 3
R¹, R², R³ : indépendamment les uns des autres H ou un reste hydrocarboné cyclique ou polycyclique, linéaire, ramifié, saturé ou insaturé avec de 0 à 10 hétéroatomes du groupe O, N, S et au total de 1 à 30 atomes de C,
X : un groupe de la formule globale CₙH₂ₙ,
n : un nombre entier ≥ 3,
à condition que n > 3 et X contient au moins un atome C tertiaire et/ou quaternaire dans le cas où R¹ représente H,
(B1) de l'eau et
(B2) au moins un acide organique ou inorganique.

2. Compositions selon la revendication 1, dans lesquelles le reste hydrocarboné R¹, R² ou R³ cité représente
- un groupe alkyle en C₁-C₃₀, alcényle en C₂-C₂₀ ou alcynyle en C₂-C₂₀ éventuellement interrompu par -O-, -S- ou -N-,
- un groupe cycloalkyle en C₁-C₁₂, aryle en C₁-C₁₀, hétéroaryle en C₁-C₉ éventuellement substitué par un groupe alkyle en C₁-C₆, alcényle en C₂-C₆ ou alcoxy en C₁-C₆,
- un groupe aralkyle en C₇-C₁₂ ou alkaryle en C₇-C₁₂.

3. Compositions selon l'une quelconque des revendications précédentes, dans lesquelles (B) les constituants B1 et B2 sont présents dans la forme d'un mélange.

4. Compositions selon au moins l'une des revendications précédentes, **caractérisées en ce que** le constituant (A) présente une teneur en groupes polyéther de 50 à 90 %.

5. Compositions selon au moins l'une des revendications précédentes, **caractérisées en ce que** le constituant (A) présente des groupes terminaux de la structure NR¹-X-Si(OR²)ₘR³₍₃₋ₘ₎, avec m = 1, 2 ou 3, X représentant un groupe de la formule globale CₙH₂ₙ, avec n > 3 et R¹, R² et R³ ayant la signification indiquée dans la revendication 1 ou 2.

6. Compositions selon au moins l'une des revendications précédentes 1 à 5, **caractérisées en ce que** le dérivé de polyéther silane-terminé contenu présente des groupes terminaux de la structure NR¹-X-Si(OR²)ₙR³₍₃₋ₘ₎, avec m = 1, 2 ou 3, X représentant un groupe de la formule globale CₙH₂ₙ, avec 3 ≤ n ≤ 20 et au moins un atome de C tertiaire et/ou au moins un atome de C quaternaire et R¹, R² et R³ ont les significations indiquées dans la revendication 1 ou 2.

7. Compositions selon au moins l'une des revendications 1 à 5, **caractérisées en ce que** le dérivé de polyéther silane-terminé contenu présente des groupes terminaux de la structure NR¹-X-Si(OR²)ₘR³₍₃₋ₘ₎, avec m = 1, 2 ou 3, X étant un groupe de la formule globale CₙH₂ₙ avec n>3,
R¹ représente un reste alkyle, cycloalkyle, phényle, benzyle ou carbamate et R² et R³ ont la signification indiquée dans la revendication 1 ou 2.

8. Compositions selon au moins l'une des revendications précédentes, **caractérisées en ce que** le constituant (B) présente des mélanges contenant de l'eau et des acides organiques/inorganiques dans des rapports de quantité en poids de 1:0,01 à 1:40.

9. Compositions selon au moins l'une des revendications précédentes, **caractérisées en ce que** sont contenus comme autres constituants
(C) de 5 à 70 % de diluant et/ou
(D) de 10 à 90 % de charge et/ou
(E) de 0 à 10 % d'additifs classiques.

10. Compositions selon au moins l'une des revendications précédentes, **caractérisées en ce que** sont contenus comme autres constituants
(C) de 15 à 50 % de diluant et/ou
(D) de 20 à 70 % de charge et/ou
(E) de 0 à 8 % d'additifs classiques.

11. Compositions selon la revendication 9 ou 10, dans lesquelles les additifs du constituant (E) appartiennent aux groupes des colorants, des pigments, des arômes, des stabilisateurs, des retardateurs, des émulsionnants.

12. Utilisation des compositions selon au moins l'une des revendications 1 à 11 pour la préparation de matières dentaires.

13. Utilisation des compositions selon au moins l'une des revendications 1 à 11 pour le moulage.

14. Récipient contenant au moins une composition selon au moins l'une des revendications 1 à 11.

15. Dispositif de mélange contenant au moins une composition selon au moins l'une des revendications 1 à 11.
